# EUROPEAN PATENT APPLICATION

(11) **EP 1 530 969 A1**
(43) Date of publication of application: **18.05.2005**
(21) Application number: 04078357.3
(22) Date of filing: 10.05.2001
(51) Int. Cl.: A61K 33/24, A61K 31/337, A61K 31/7068, A61P 35/00, A61K 45/06

(54) **Combination chemotherapy**

(30) Priority: 18.05.2000 GB 0011903
(62) Divisional of application: 01925764.1
(71) Applicant: ANORMED INC., Langley, British Columbia V2Y 1N5 (CA)
(72) Inventor: Smith, Mark Peart, Cheshire, SK 10 4TG (GB); Stephens, Trevor Charles, Cheshire, SK10 4TG (GB)
(74) Representative: W.P. Thompson & Co.

(57) **Abstract**

The invention relates to a pharmaceutical composition, a therapeutic combination product and a kit comprising a sterically hindered platinum coordination compound and a non-platinum based anti-cancer agent and a pharmaceutically-acceptable carrier or diluent; to the use thereof for the manufacture of a medicament for inhibiting the growth of tumour cells in a human afflicted therewith; to a method of inhibiting the growth of tumour cells in a human afflicted therewith which comprises administering to such human an effective tumour cell growth inhibiting amount of such a therapeutic combination product or of a pharmaceutical composition of the invention; in particular when the sterically hindered platinum coordination compound is (SP-4-3)-(cis)-am-minedichloro-[2-methylpyridine]platinum (II), or a pro-drug thereof, and the non-platinum based anti-cancer agent is selected from Taxol Gemcitabine, Navelbine, Doxil, 5-FU and Taxotere.

## Description

This invention relates to a therapeutic combination product and also to a kit comprising a sterically hindered platinum coordination compound in combination with a non-platinum based anti-cancer agent, and to a pharmaceutical composition comprising a sterically hindered platinum coordination compound and a non-platinum based anti-cancer agent and a pharmaceutically-acceptable carrier or diluent. The invention also relates to a method of inhibiting the growth of tumour cells in a human afflicted therewith which comprises administering to such human an effective tumour cell growth inhibiting amount of such a therapeutic combination product or of a pharmaceutical composition of the invention. The invention also relates to the use of a sterically hindered platinum coordination compound in combination with a non-platinum based anti-cancer agent for the manufacture of a medicament for inhibiting the growth of tumour cells in a human afflicted therewith.

Cisplatin, or cis-dichlorodiammine platinum (II), has been used for many years as a chemotherapeutic agent in the treatment of various human solid malignant tumours, and possesses a very broad spectrum of activity. Although antitumour activity of cisplatin was reported in tumours such as refractory testicular teratoma and ovarian cancer, the incidence of severe side effects such as kidney damage, and nausea & vomiting was initially unacceptable. These side effects have been alleviated to some extent (e.g., nephrotoxicity using hyperhydration with isotonic saline, nausea & vomiting with the advent of 5-hydroxytryptamine type 3 (5-HT₃) antagonists), but there has been a major synthetic drive to develop platinum analogues possessing a more acceptable toxicity profile. More recently, platinum analogues such as carboplatin (*cis*-diammine-1,1-cyclobutane dicarboxylate platinum (II)), have shown efficacy as chemotherapeutic agents in the treatment of various human solid malignant tumours. Carboplatin, although less neurotoxic and less nephrotoxic than cisplatin, still shows nephrotoxicity, and is also significantly more myelotoxic than cisplatin.

However, although platinum based agents such as cisplatin and carboplatin have been widely used as chemotherapeutic agents in humans, they are not therapeutically effective in all patients or against all types of solid tumours. Additionally, tolerance or resistance of tumours to cisplatin represents a major impediment to successful treatment. Such resistance is often considered as either intrinsic (i.e. present at the onset of treatment) or acquired (i.e. occurs during courses of chemotherapy). Typical of tumours exhibiting intrinsic resistance to cisplatin are colorectal and non-small cell lung cancers while acquired resistance is often observed in patients with ovarian or small cell lung cancers.

As mentioned above, a key goal in the development of improvements over cisplatin/carboplatin has been to overcome drug resistance. The observation that agents in which the ammine ligands were replaced by a diaminoclycohexane (DACH) group were active against cisplatin resistant cell lines led to the development of a range of such agents. Amongst these, oxaliplatin has shown activity in advanced colorectal cancer both as a single agent and in combination with 5-fluorouracil (5-FU). Oxaliplatin however produces a curious pattern of neurotoxicity, including facial dysaesthesia which may be provoked by exposure to cold. Peripheral sensory neuropathy may also occur. Despite such neurotoxicity, the drug does not cause significant nephrotoxicity, and oxaliplatin represents an advance in the treatment of advanced colorectal cancer.

Thus, despite some advances in overcoming cisplatin resistance and in the alleviation of toxicity side-effects, the field of platinum anti-cancer agents lacks an agent capable of demonstrating antitumour activity in a range of clinically important cancers, whilst also possessing an acceptable toxicity profile. Furthermore, the outcome of current chemotherapy regimens in cancer patients is currently unsatisfactory. A number of different approaches to overcoming resistance to cisplatin are being studied, and the prevention of platinum-related toxicity is also of considerable interest.

Platinum-based chemotherapy is frequently given with one or more different agents in the treatment of various cancers. For example, for most SCLC patients, cisplatin and etoposide are the initial choice for combination chemotherapy. Carboplatin can be substituted for cisplatin without loss of efficacy. In limited-stage disease, the addition of irradiation to standard chemotherapy results in a modest improvement in survival, while in extensive-stage disease, radiotherapy does not improve survival, but does play an extremely important palliative role. A further combination of interest is cisplatin plus paclitaxel, which is used in the treatment of advanced ovarian cancer.

Thus, combinations may offer improved efficacy, but the unacceptable toxicity profile of previous platinum-based anti-cancer agents and other difficulties, such as scheduling issues and need for fluid hydration, still makes many such combinations unsatisfactory. There is therefore a need for increasing the efficacy of the tumour cell growth inhibiting activity of known chemotherapeutic combinations involving platinum complexes and/or providing a means for the use of lower dosages of chemotherapeutic agents to reduce the potential or adverse toxic side effects to the patient. Although desirable, synergy is not often observed in combination approaches to cancer treatment. The therapeutic combination product, pharmaceutical composition, use and methods of this invention fill such a need.

(SP-4-3)-(*cis*-amminedichloro-[2-methylpyridine]platinum (II), hereinafter ZD0473, is a novel sterically hindered platinum complex developed to overcome acquired or *de novo* resistance to cisplatin. ZD0473 is described in US 5,665,771, where it is stated that: "complexes of the invention may be administered alone or in combination with another chemotherapeutic agent such as cis-platin, either as a single treatment or course of treatment or as part of combined therapy with other pharmaceuticals to overcome or diminish side-effects or to improve bio-availability, or in combination with other therapies such as radiation treatment". Nowhere in US 5,665,771 does it state that use of any complex of the invention therein with other treatments will produce surprisingly beneficial effects.

Interim results of the monotherapy Phase I trial involving ZD0473 were presented at the 35^{th} annual American Society of Clinical Oncologists meeting in Atlanta in May 1999, indicating that the dose-limiting toxicity of ZD0473 was bone marrow suppression, with no evidence of kidney or nerve toxicity.

For ZD0473, both neutropenia and thrombocytopenia are observed but recovery is quite rapid and patients who have not been heavily pre-treated can be treated at 3-weekly intervals. Cumulative anaemia and thrombocytopenia are being investigated. There has been no clinically relevant evidence of peripheral neurotoxicity, ototoxicity or renal toxicity. Ototoxicity can be problematic, especially when using cis-platin, as it is usually irreversible. The recommended dose and schedule for phase II studies was initially 120 mg/m² every 3 weeks. Patients who have been heavily pre-treated with cytotoxic agents which damage bone marrow stem cells may require dose reductions and may not be able to tolerate the drug every 3 weeks. However, there are patients who will tolerate higher doses, e.g. 150 mg/m² (the current recommended dose) or higher. Evidence of antitumour activity has been observed in patients with a variety of malignancies including small & non-small cell lung cancer, mesothelioma, head and neck, breast and ovarian cancers. ZD0473 is being studied to determine the maximum levels of the drugs that can be safely given to patients, to examine how the drugs are handled by the human body and to determine the nature of the dose-limiting toxicity.

Unexpectedly, and surprisingly, we have now found that the particular compound ZD0473 used in combination with a non-platinum based anti-cancer agent, produces significantly better anti-cancer (anti-cell proliferation) effects than ZD0473, or the non-platinum based anti-cancer agents used alone. It is to be understood that also covered in the definition of the particular compound ZD0473 are pro-drugs which produce the active ZD0473 species in-vivo (for example the Pt(IV) tri-hydroxy, mono-chloro, mono-ammine prodrug of ZD0473). Preferred embodiments of the invention relate to the sterically hindered platinum coordination compound (SP-4-3)-(*cis*-amminedichloro-[2-methylpyridine]platinum (II). We believe that ZD0473 (and similar sterically hindered platinum (Pt(II) & Pt(IV)) complexes in which the 2-methylpyridine ligand of ZD0473 is replaced with another sterically hindered substituted amine as described in US 5,665,771. The relevant definitions and Examples of US 5,665,771 are hereby incorporated by reference) possesses the highly desirable activity and side-effect profile which has been lacking in current platinum-cytotoxic combination regimes, rendering it particularly suitable for use in combination with non-platinum based anti-cancer agents. Particularly beneficial for combination use is ZD0473's combination of a favourable myelosuppression profile with a lack of significant neurotoxicity and nephrotoxicity.

Thus, it has been found that there is a therapeutic benefit when a sterically hindered platinum coordination compound such as ZD0473 is administered in combination with other non-platinum-based chemotherapeutic agents, thereby potentially increasing the tumour cell growth inhibiting activity compared to the use of each of the components individually. It has also now been found that such a benefit may result in a need for lower doses of such a sterically hindered platinum coordination compound and/or of the other non-platinum based anti-cancer agent compared to the use of each of the components individually. In certain cases, higher doses of each, or both of, components may be delivered when, for example, the combination imparts a protective effect on normal (non-tumour) cells, thereby permitting higher doses to be tolerated. The particularly favourable toxicity profile (including favourable neurotoxicity and nephrotoxicity) of ZD0473 permits it to be used beneficially with other non-platinum based anti-cancer agents which might otherwise be limited in their combination potential because of their own toxicity profile (for example, Taxol doses may be limited in combination with earlier platinum agents by virtue of Taxol's own neurotoxicity). The benefits of the present invention may be demonstrated by suitable in-vitro experiments or improved in-vivo performance (such as, for example, improved toxicity or evidence of a platelet sparing effect compared with mono-therapy or with combinations using earlier platinum agents).

As stated above, platinum based drugs such as cisplatin and carboplatin are widely used in the treatment of solid tumours such as cancers of the lung, ovary and testes. Many of these tumours initially respond to platinum based therapy but, in most cases, the tumours become resistant and the disease recurs. ZD0473 was targeted to overcome this resistance against platinum drugs and thereby provide an extended spectrum of anti-cancer activity. *In-vivo* studies in human tumour xenografts have shown ZD0473 activity at least comparable and in some cases superior to that of cisplatin. Activity was retained versus a cisplatin-resistant CH1 ovarian tumour xenograft, even when progressing on cisplatin treatment. Against murine tumours activity was retained when the drug was administered orally and some activity was seen against the acquired cisplatin resistant ADJ/PC6cisR tumour which is totally resistant to other platinum agents.

ZD0473 has been studied *in-vitro* in human tumour cell lines displaying a range of sensitivity to platinum agents and the corresponding cell line with acquired resistance to cisplatin and demonstrated the ability to overcome platinum resistance due to a variety of mechanisms. Moreover, good retention of activity was observed in cell lines manipulated to possess relatively high levels of either glutathione or metallothioneins. Interestingly, in addition to reduced susceptibility to inactivation by glutathione, ZD0473 also showed the ability to overcome resistance in the 41McisR ovarian line in which resistance to cisplatin is due to reduced uptake. Investigations into the nature of interactions between ZD0473 and DNA demonstrated differences in the sequence specificity of DNA adduct formation and the presence of a unique ZD0473 binding site.

Thus, a further benefit of the present invention is that a sterically hindered platinum coordination compound such as ZD0473 may be administered in combination with other non-platinum-based chemotherapeutic agents, to produce therapeutically beneficial tumour cell growth inhibiting activity in tumours and cell-lines that are resistant and lacking in sensitivity to other platinum-based (non-sterically hindered) coordination compounds. Combination therapies involving platinum-based coordination compounds that might have been ineffective are now therefore possible with a sterically hindered platinum coordination compound such as ZD0473. Thus, second-line treatment of patients who might previously have been considered unlikely to benefit from combination therapy involving a platinum-based coordination compound may now be treated in combination therapy using a sterically hindered platinum coordination compound such as ZD0473.

Accordingly, the present invention provides a therapeutic combination product comprising a sterically hindered platinum coordination compound and a non-platinum based anti-cancer agent suitable for administration simultaneously, sequentially or separately. Preferably the components of the combination are administered simultaneously and/or separately, so as to deliver both agents in-vivo at the same time (i.e. concomitant exposure). The skilled man will be readily able to ascertain whether the components are present in-vivo at the same time using standard techniques.

According to a further aspect of the present invention there is provided a kit comprising a sterically hindered platinum coordination compound and a non-platinum based anti-cancer agent mentioned herein.

According to a further aspect of the present invention there is provided a kit comprising:
a) a sterically hindered platinum coordination compound in a first unit dosage form;
b) a non-platinum based anti-cancer agent mentioned herein in a second unit dosage form; and
c) container means for containing said first and second dosage forms.

According to a further aspect of the present invention there is provided a kit comprising:
a) a sterically hindered platinum coordination compound together with a pharmaceutically acceptable excipient or carrier, in a first unit dosage form;
b) a non-platinum based anti-cancer agent mentioned herein, together with a pharmaceutically acceptable excipient or carrier, in a second unit dosage form; and
c) container means for containing said first and second dosage forms.

According to a further aspect of the invention there is provided a pharmaceutical composition which comprises a sterically hindered platinum coordination compound and a non-platinum based anti-cancer agent a platinum anti-tumour agent mentioned herein, in association with a pharmaceutically acceptable excipient or carrier.

According to a further aspect of the present invention there is provided a combination product comprising a sterically hindered platinum coordination compound and a non-platinum based anti-cancer agent mentioned herein for use as a medicament, and further for use in a method of treatment of a human or animal body by therapy.

According to a further aspect of the present invention there is provided a pharmaceutical composition as defined herein for use as a medicament, and further for use in a method of treatment of a human or animal body by therapy. According to a further aspect of the present invention there is provided the use of a sterically hindered platinum coordination compound in combination with a non-platinum based anti-cancer agent as defined herein in the manufacture of a medicament for use in the production of an anti-cancer effect in a warm-blooded animal such as a human.

According to a further aspect of the present invention there is provided the use of a therapeutic combination product, or a pharmaceutical composition as defined herein in the manufacture of a medicament for use in the production of an anti-cancer effect in a warm-blooded animal such as a human. According to a further aspect of the present invention there is provided a method for producing an anti-cancer effect in a warm-blooded animal such as a human which comprises the administration of a sterically hindered platinum coordination compound in combination with a non-platinum based anti-cancer agent as defined herein.

According to a further aspect of the present invention there is provided a method for producing an anti-cancer effect in a warm-blooded animal such as a human which comprises the administration of a therapeutic combination product, or a pharmaceutical composition as defined herein.

In each of the above aspects of the invention, the preferred sterically hindered platinum coordination compound is ZD0473.

According to a further aspect of the present invention there is provided a combination treatment comprising the administration of an effective amount of ZD0473, optionally together with a pharmaceutically acceptable excipient or carrier, and the simultaneous, sequential or separate administration of an effective amount of a non-platinum based anti-cancer agent as defined herein to a warm-blooded animal such as a human in need of such therapeutic treatment.

According to another aspect of the present invention the effect of a method of treatment of the present invention is expected to be at least equivalent to the addition of the effects of each of the components of said treatment used alone, that is, of each of ZD0473 and the preferred non-platinum based anti-cancer agents mentioned herein. Such additivity is surprisingly beneficial if both agents of the combination might target the same tumour cells and/or the same stage in cell cycling.

According to another aspect of the present invention the effect of a method of treatment of the present invention is expected to be greater than the addition of the effects of each of the components of said treatment used alone, that is, of each of ZD0473 and the preferred non-platinum based anti-cancer agents mentioned herein. Such synergy is surprisingly beneficial as the MTD levels are less than those for each agent individually. Such benefit can be observed when normal cells can tolerate higher doses (i.e. show greater antagonism) of either or both component better than tumour cells.

A combination treatment as defined herein may be applied as a sole therapy or may involve surgery and/or ionising radiation treatment, in addition to a combination treatment of the invention: Surgery may comprise the step of partial or complete tumour resection, prior to, during or after the administration of the combination treatment described herein.

Such combination treatments of the present invention are expected to be useful in the prophylaxis and treatment of a wide range of disease states including cancer, such as, for example, lung cancer (such as SCLC or NSCLC), mesothelioma, ovarian, breast, cervix/uterus, bladder, prostate, testicular, pancreatic, head & neck and liver cancer, Kaposi's sarcoma, lymphoma (NHL), leukaemia, and other cell-proliferation diseases such as, for example, psoriasis, arthritis and fibrosis (for example of the lung). In particular, such combination treatments of the invention are expected to slow advantageously the growth of primary and recurrent solid tumours of, for example, but not limited to, GI/stomach, colon, rectal, breast, prostate, lungs, testes, skin, bone & sarcoma and kidney.

In particular embodiments of the present invention the ionising radiation employed may be X-radiation, γ-radiation or β-radiation. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. It is most likely that all of these factors effect a broad range of damage on DNA, on the precursors of DNA, on the replication and repair of DNA and on the assembly and maintenance of chromosomes. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

By the term "sterically hindered platinum coordination compound" is meant any tumour cell growth inhibiting sterically hindered platinum coordination compound described and encompassed in US 5,665,771 (the compounds of which, and their preparation, is incorporated herein by reference). The preferred sterically hindered platinum coordination compound is ZD0473.

By "a non-platinum based anti-cancer agent" we mean a compound with anti-cancer and/or anti-cell proliferation activity that does not contain platinum, in particular, a compound or drug selected from the following classes :-
1. A compound of the camptothecin analogue class, i.e. any tumour cell growth inhibiting compound which is structurally related to camptothecin, and inhibits topoisomerase I; or a compound of the podophyllotoxin analogue class which inhibits topoisomerase II; or is a compound of the camptothecin analogue class which is an inhibitor of both topoisomerase I and II. Suitable compounds of the camptothecin analogue class include, but are not limited to, pure topoisomerase I inhibitors such as Topotecan, Irinotecan, 9-Aminocamptothecin, Rubitecan and Exatecan (DX-8951f); mixed topoisomerase I and topoisomerase I I inhibitors such as XR-5000 and XR-11576; and suitable compounds of the podophyllotoxin analogue class which are pure topoisomerase II inhibitors include, but are not limited to, Etoposide and Teniposide. Such compounds also include, but are not limited to, any tumour cell growth inhibiting camptothecin analogue claimed or described in WO 93/09782 and the references cited therein (which are hereby incorporated herein by reference). The preparation of Topotecan (including pharmaceutically acceptable salts, hydrates and solvates thereof) as well as the preparation of oral and parenteral pharmaceutical compositions comprising topotecan and an inert, pharmaceutically acceptable carrier or diluent, is extensively described in U.S. Patent 5,004,758 and European Patent Application Publication Number EP 0,321,122.
2. A Taxane, such as Taxol (paclitaxel) or Taxotere (docetaxel).
3. A Growth-factor receptor inhibitor such as a protein-kinase inhibitor, such as a class I tyrosine kinase inhibitor, such as Iressa (ZD1839), and inhibitors of growth factor function (such growth factors include for example platelet derived growth factor and hepatocyte growth factor and such inhibitors include growth factor antibodies, growth factor receptor antibodies and serine/threonine kinase inhibitors). Also included are inhibitors of cell cycle kinases such as CDK-2, CDK-4 and CDK-6.
4. An Anti-metabolite such as 5-FU, S1, UFT, Capecitabine; a thymidylate synthase inhibitor such as Tomudex or ZD9331, or LY231514 (MTA, pemetrexed) or Gemcitabine, or an antifolate such as Methotrexate.
5. A vinca alkaloid such as Vinolrebine (Navelbine), Vincristine, Vinblastine or Vindesine.
6.. An Anti-angiogenic compound such as described in International Patent Application Publication Nos. WO 97/22596, WO 97/30035, WO 97/32856, WO 98/13354, WO 00/21955 and WO 00/47212.
7. An Alkylating agent such as Melphalan, Cyclophosphamide, Ifophamide or a Nitroso-urea, such as Carmustine or Lomustine.
8. An Anthracyclin such as Doxrubicin, Epiribicin, Idarubicin or Doxil.
9. An anti-HER-neu compound, such as Herceptin.
10. A cytostatic agent such as an antioestrogen (for example tamoxifen,toremifene, raloxifene, droloxifene, iodoxyfene), a progestogen (for example megestrol acetate), an aromatase inhibitor (for example anastrozole, letrazole, vorazole, exemestane), an antiprogestogen, an antiandrogen (for example flutamide, nilutamide, bicalutamide, cyproterone acetate), LHRH agonists and antagonists (for example goserelin acetate, luprolide), an inhibitor of testosterone 5α-dihydroreductase (for example finasteride) and an anti-invasion agent (for example metalloproteinase inhibitors like marimastat and inhibitors of urokinase plasminogen activator receptor function).
11. Antimitotics, natural and synthetic.
12. Interleukins and cytokines such as TNF.
13. Vaccines.
14. Uptake/efflux modulators such as mdr2
15. Rescue agents.
16. Ca antagonists.

It will be appreciated that other platinum agents (such as BBR3464 or oxaliplatin) with a different mode of action or useful profile may also be used with ZD0473.

All of the such listed non-platinum based anti-cancer agents are available commercially and/or can be prepared by conventional techniques including those described in the above-listed references. Other formulations, such as polyglutamate polymers (biodegradable polymers with a cytotoxic agent attached) and liposomal formulations of the above non-platinum based anti-cancer agents are also included.

Preferred non-platinum based anti-cancer agents are those from classes 1, 2, 3, 4, 5, 6 and 8 above, especially from classes 2, 3, 5 and 8.

Of the above non-platinum based anti-cancer agents, those particularly preferred for use in the present invention are Taxol, Topotecan, Gemcitabine, Navelbine, Doxil and 5-FU.

Thus, the preferred components for use in the various embodiments of the present invention are the sterically hindered platinum coordination compound ZD0473, and a non-platinum based anti-cancer agents selected from Taxol or Topotecan or Gemcitabine or Navelbine or Doxil or 5-FU. Also preferred is ZD0473 and Taxotere.

As a further feature of the invention there are provided triplet combinations (for example, ZD0473 plus Taxol plus Iressa or Gemcitabine; or ZD0473 plus an anthracyclin plus a hormonal agent) in which agents with different modes of action are employed.

As a further feature of the invention there are provided sequential doublet combinations (for example, ZD0473 plus Taxol, followed later by, for example, ZD0473 plus Gemcitabine) in which a different second agent (with a different mode of action) is used later. Sequential triplet combinations may be possible if the toxicity profile of the agents in combination permits.The term "anti-cancer effect" includes inhibiting the growth of tumour cells and/or the cytotoxic killing of tumour cells which are sensitive to a combination product, composition and treatment of the present invention.

The term "inhibiting the growth of tumour cells" as used herein is meant the inhibition of the growth of tumour cells which are sensitive to the method of the subject invention. Anti-tumour effects of the use and method of treatment of the present invention include but are not limited to, inhibition of tumour growth, tumour growth delay, regression of tumour, shrinkage of tumour, increased time to regrowth of tumour on cessation of treatment, slowing of disease progression. It is expected that when a use and method of treatment of the present invention is administered to a warm-blooded animal such as a human, in need of treatment for cancer involving a solid tumour, said use and method of treatment will produce an effect, as measured by, for example, one or more of: the extent of the anti-tumour effect, the response rate, the time to disease progression and the survival rate. Preferably such treatment also leads to the regression of tumour growth, i.e. the decrease in size of a measurable tumour. Most preferably, such treatment leads to the complete regression of the tumour. The present invention may be used, for example in elderley, paediatric or renally or hepatically impaired patients.

By the term "effective tumour cell growth inhibiting amount" and "effective amount" as used herein is meant a course of therapy which will result in inhibiting the growth of tumour cells sensitive to such therapy in a human afflicted therewith. Preferably, such course of therapy will result in the administration of a lower dose of a sterically-hindered platinum coordination compound, and/or of the non-platinum based anti-cancer agent, than is required when such compound is administered as the sole chemotherapeutic agent. Preferably, such course of therapy will result in enhancement of the tumour cell growth inhibiting efficacy of the non-platinum based anti-cancer agent and/or the sterically-hindered platinum coordination compound as compared to when such compound is administered as the sole chemotherapeutic agent. Particularly preferred is a classical synergistic effect in which the combined effect of both components is greater/superior to that anticipated from dosing both components together (i.e. greater than an additive effect). It will be appreciated that the actual preferred course of therapy will vary according to, inter alia, the mode of administration of the compounds, the particular formulation of the compounds being utilized, the mode of administration, the particular tumour cells being treated and the particular host being treated. The optimal course of therapy for a given set of conditions can be ascertained by those skilled in the art using conventional course of therapy determination tests and in view of the information set out herein.

The pharmaceutical composition of this invention contains both a sterically hindered platinum coordination compound and a non-platinum based anti-cancer agent as defined herein, as well as a pharmaceutically acceptable carrier or diluent. The appropriate pharmaceutically acceptable carriers and diluents to be utilized in the composition of the invention are well known to those skilled in the art of formulating compounds into pharmaceutical compositions. The pharmaceutical composition of the invention will be in a form suitable for parenteral or oral administration. Such composition may be formulated for intravenous infusion or injection in numerous ways well known to those skilled in the art with pharmaceutically acceptable carriers, for example with saline. Preferably, such pharmaceutical composition is in the form of a freeze-dried mixture of the two active ingredients in a unit dosage form, prepared by conventional techniques, which can be reconstituted with water or other suitable infusion liquid at the time of administration.

It will be recognized by one skilled in the art that the content of the active ingredients in the pharmaceutical composition of this invention may vary quite widely depending upon numerous factors, such as, the desired dosage and the pharmaceutically acceptable carrier being employed. For administration, in the pharmaceutical composition and other aspects of the invention, the administration dose ratio of the sterically hindered platinum coordination compound to the non-platinum based anti-cancer agent will usually be in the range 10:1 to 1: 1000 by weight. Preferably, the combination product and pharmaceutical composition of the invention will contain from 50 mg to 600 mg of the sterically-hindered platinum coordination compound per unit dosage form, and from 5 mg to 5,000 mg of the non-platinum based anti-cancer agent per unit dosage form. Mannitol and/or sodium chloride may preferably be included in conventional amounts. Physiological pH of injectables or infusion drug combinations will be established by inclusion of buffering agents as is known in the pharmaceutical formulation art.

The combination product and compositions described herein may be in a form suitable for oral administration, for example as a tablet or capsule (enteric coated as appropriate), for example as a powder or solution, for parenteral injection (including intravenous, subcutaneous, intramuscular, intravascular, intraperitoneal or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD0473 of the combination treatment may be delivered endoscopically, intratracheally, intralesionally, percutaneously, intravenously, subcutaneously, intraperitoneally or intratumourally. In general the compositions described herein may be prepared in a conventional manner using conventional excipients. The compositions of the present invention are advantageously presented in unit dosage form.

The size of the dose of each therapy which is required for the therapeutic or prophylactic treatment of a particular disease state will necessarily be varied depending on the host treated, the route of administration and the severity of the illness being treated. Accordingly, the optimum dosage may be determined by the practitioner who is treating any particular patient. For example, it may be necessary or desirable to reduce certain doses of the components of the combination treatments in order to reduce toxicity. By the term "administering" or "administered" as used herein is included parenteral and/or oral administration. By "parenteral" is meant intravenous, subcutaneous, intramuscular or intraperitoneal or infusion administration. It will be understood that the actual preferred method and order of administration will vary according to, inter alia, the particular formulation/s being utilized, the particular tumour cells being treated, and the particular host being treated. The optimal method and order of administration for a given set of conditions can be ascertained by those skilled in the art using conventional techniques and in view of the information set out herein.

In the method of the subject invention, the sterically-hindered platinum coordination compound can be administered in the same manner as in prior clinical practice. More specifically, slow intravenous infusion is the usual method of choice for ZD0473. For promoting diuresis when using ZD0473, the incorporation of mannitol in a dextrose/saline solution is the preferred carrier. The protocol can also include prehydration of the patient by administration of a dextrose/salin solution. In the method of the subject invention, the dose schedule of the sterically-hindered platinum coordination compound may be on the basis of from about 25 to about 500 mg per square meter (mg/m²) of body surface area per course of treatment, for example approximately 0.4-10mg/kg in a human.. For the method of the subject invention utilizing ZD0473, the preferred dosage of ZD0473 would be a single dose of from about 30 to about 250 mg/m² of ZD0473 at the end of a one to five consecutive day course of treatment. Infusions of the sterically-hindered platinum coordination compound may be given one to two times weekly, and the weekly treatment repeated several times unless side effects provide a contraindication. A unit dosage form such as a tablet or capsule will usually contain, for example 50-600mg of active ingredient. The use of fractionate doses of ZD0473 (for example 30mg/m²/day over 5 days rather than 150 mg/m² in one day) may be usefully employed, for example in conjunction with daily radiation treatment. Other conventional practices may be employed in conjunction with the administration, for example supportive concomitant therapy, GCSF/EPO. The non-platinum based anti-cancer agents may be dosed according to known routes of administration and dosages, as illustrated, but not limited, by the following examples.

For example, in the method of the subject invention, the parenteral administration of a compound of the camptothecin analogue class, the course of therapy generally employed is from about 0.1 to about 300.0 mg/m² of body surface area per day for about one to about five consecutive days, more preferably, from about 0.1 to about 100 mg/m² of body surface area per day for about one to five consecutive days. Most preferably, the course of therapy employed for Topotecan is from about 1.0 to about 2.0 mg/m² of body surface area per day for about one to five consecutive days. Preferably, the course of therapy is repeated at least once at about a seven day to about a twenty-eight day interval (from the date of initiation of therapy) depending upon the initial dosing schedule and the patient's recovery of normal tissues. Most preferably, the course of therapy continues to be repeated based on tumour response.

Preferably, the parenteral administration of a compound of the camptothecin analogue class will be by short (e.g. 30 minutes) or prolonged (e.g. 24 hour) intravenous infusion. More preferably, the compound the camptothecin analogue class will be administered by a 30 minute intravenous infusion. A preferred course of parenteral therapy with Topotecan is an initial course of therapy of 1.5 mg/m² of body surface area per day administered by short intravenous infusion for one to five consecutive days in previously non-treated or lightly pretreated patient. For a heavily pretreated patient an initial course of Topotecan therapy of 1.0mg/m² of body surface area per day is administered by short intravenous infusion for one to five consecutive days. When the patient has recovered sufficiently from the drug-related effects of the initial course, an additional course of therapy of at least the same dose per day as the initial dose is administered by short intravenous infusion for one to five consecutive days, to be repeated based on tumour response.

In the method of the subject invention, for oral administration of a compound the camptothecin analogue class, the course of therapy generally employed is from about 1.0 to about 500.0mg/m² of body surface area per day for about one to five consecutive days. More preferably, the course of therapy employed for Topotecan is from about 1.5 to about 5.0 mg/m² of body surface area per day for about one to five consecutive days. Preferably, the course of therapy is repeated at least once at about a seven day to about a twenty-eight day interval (from the date of initiation of therapy) depending upon the initial dosing schedule and the patient's recovery of normal tissues. Most preferably, the course of therapy continues to be repeated based on tumour response.

For example, Taxol (paclitaxel) may be administered as an infusion over a period of about 24 hours at a dose of 135-200mg/m² every 3 weeks. Alternatively, for example, Taxol may be administered as an infusion over a period of about 3 hours at a dose of 135-225mg/m² every 3 weeks. Alternatively, for example, Taxol may be administered as an infusion over a period of about 1 hour at a dose of 80-100mg/m² every week for a number of weeks. Alternatively, for example, Taxol may be administered as an infusion over a period of about 1 hour at a dose of 200mg/m² every 3 weeks. Alternatively, for example, Taxol may be administered as an infusion over a period of about 96 hours at a dose of 120-140mg/m² every 3 weeks.

For example, Docetaxel may be dosed in according with known routes of administration and dosages. For example Docetaxel may be administered as an infusion over a period of 1 hour at a dose of 55-100mg/m² every 3 weeks.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

As stated above, the components of the invention may be administered in a simultaneous, sequential or separate manner. Preferably the administration is simultaneous and/or sequential, provided that each component is thereby present in-vivo at a therapeutically effective concentration at the same time. Thus, depending for example on the pharmaco-kinetics of the individual components and the administration route, the individual agents may be dosed separately (with a gap of, for example, 10-60 minutes), and this may effectively achieve an in-vivo profile for the combination equivalent, or similar, to that achieved by simultaneous administration.

Similarly, depending for example on the pharmaco-kinetics of the individual components, the components of the invention may be administered in a range of repeat cycles. For example, once a week for a number of weeks; once daily over five days and repeated over a number of weeks, or continuously on a daily basis for a number of weeks. The administration may be via bolus oral and/or iv administration or via continuous iv infusion, depending on the particular combination chosen and the nature of the repeat cycle. Furthermore, each component of the combination may be administered using the same or different repeat cycles. Thus, for example, the non-platinum based anti-cancer agent may be administered once a week, whilst ZD0473 is administered daily over 5-days, and the cycle then repeated over a number of weeks.

### Experimental & Results

The following, non-limiting, *in-vitro* tests combining ZD0473 with Taxol (paclitaxel) were used to demonstrate the combinations of the present invention. Details of cultures used and other experimental details may be found in Holford et al, Br.J.Cancer, 1998, 7793, 366-373 and K.E.Pestell et al, Molecular Pharmacology, 57 : 503-511, 2000 (the relevant practical details of which are hereby incorporated by reference).

Four human ovarian carcinoma cell lines were used: two parent cisplatin-sensitive lines, CH1 and A2780; a 15-fold acquired cisplatin resistant subline, A2780cisR; and an A2780 subline stably transfected with the E6 human papillomavirus gene thereby disabling the wild-type p53 function of the parental line, A2780 E6. Growth inhibition (using the sulforhodamine B assay) was determined for ZD0473 alone, and combined either simultaneously or administered sequentially (following washing) for 24 hours with paclitaxel. The effect of the drug combinations was analysed using median effect analysis.

ZD0473 alone (96 hour continuous exposure) exhibited potent growth inhibition against these cell lines with IC₅₀ values (µM) of 3.7 for A2780, 15.6 for A2780cisR, 8.6 for A2780E6 and 3.3 for CH1. Thus, A2780cisR was only 4.2-fold cross-resistant to ZD0473 in these studies. Paclitaxel alone also conferred potent growth inhibition with IC₅₀ values in nM of 2.5 for A2780, 3.5 for A2780cisR, 20.8 for A2780E6 and 3.1 for CH1.

When exposed concomitantly with paclitaxel the combination index (CI) values at 50% fraction affected were synergistic for all 4 cell lines (A2780 CI of 0.49, 0.61; A2780cisR CI of 0.55, 0.31; A2780E6 CI of 0.41, 0.54; CH1 CI of 0.69, 0.55).

When ZD0473 was applied for 24 hours before paclitaxel for 24 hours the effect was additive/moderately antagonistic in A2780 (CI of 1.0, 1.6) whilst some synergistic effects were obtained in the other 3 cell lines (A2780cisR CI of 0.4, 0.70; A2780E6 CI of 0.51, 0.91 and CH1 CI of 0.61, 1.30).

When cells were exposed to paclitaxel for 24 hours followed by ZD0473 for 24 hours a synergistic effect was observed in A2780cisR (CI of 0.37, 1.48) with the other cell lines giving weak synergism/an additive effect or an antagonistic effect (A2780E6 CI of 0.89, 1.02; A2780 CI of 1.1, 12.9; CH1 CI of 2.4, 2.1).

The CI figures were calculated using the method of Chou & Talalay; Adv.Enzyme Regulation, 1984, 22, 27-55. The first figure in each case using one (average) data point at each level, and the second figure incorporating all data points (including replicates) at the same data point.

These data indicate that, depending upon the cell line, the sequence in which ZD0473 and paclitaxel are administered is of importance in determining growth inhibition when combined, with most synergism being observed with simultaneous, concomitant administration, although synergy can be observed (depending on the cell-line) whatever the order of administration. Similar synergistic properties may be observed in other cell lines, such as SCLC.

The beneficial tumour cell growth inhibiting activity of the combinations of this invention may also be demonstrated in-vivo, for example in widely utilized transplantable human tumour xenograft models (for example, using a cisplatin resistant tmour) in mice (i.e. an effect greater than can be achieved with either drug used individually at its maximally tolerated dose).

The beneficial tumour cell growth inhibiting activity and interactions in toxicity of the combinations of this invention are being investigated in clinical trials. For example, in a combination trial with Gemcitabine, human cancer patients are given ZD0473 at an appropriate dose in accordance with as a 1-2 hour intravenous (iv) infusion (Day 1) and Gemcitabine at the appropriate dose as a 30-minute infusion daily for 2 days (Days 1 and 8), planned to be given every 3 weeks. On Day 1 of each cycle, ZD0473 is administered first over 1 to 2 hours, followed by a break of 30 minutes, followed by administration of Gemcitabine over 30 minutes. On Day 8, patients are given only Gemcitabine therapy over a 30-minute interval, as on Day 1. Treatment from the second, and subsequent cycles is in accordance with normal dose modification recommendations. Up to 6 different dose levels are studied, and the dose of ZD0473 and Gemcitabine escalated as follows:-

| Dose level (to be administered every 3 weeks) | ZD0473^{a} (mg/m²) Day 1 of the cycle | Gemcitabine^{a} (mg/m²) Days 1 and 8 of the cycle |
|---|---|---|
| Level 1 | 60 | 750 |
| Level 2 | 90 | 750 |
| Level 3 | 120 | 750 |
| Level 4 | 120 | 1000 |
| Level 5 | 120 | 1250 |
| Level 6 | 120 | 1500 |

| | | |
|---|---|---|
| ^{a}ZD0473 is administered over 1-2 hours, and Gemcitabine is administered over 30 minutes, beginning | | |

30 minutes after the end of ZD0473 administration on Day 1. Gemcitabine is again administered on Day 8 of each cycle. There are at least 3 patients per dose level in order to obtain 3 patients evaluable for toxicity review. If one dose-limiting toxicity DLT is encountered in one of the patients, the cohort will be extended in order to have 6 evaluable patients for toxicity review to determine if MTD has been reached. The maximum number of patients enrolled to each dose level is eight.

The definition of DLT and maximum tolerated dose (MTD), and the events defining DLT are as follows (based on CTC grading system):-
- Absolute neutrophil count (ANC) less than 0.5 x 10⁹/L associated with fever or infection
- ANC less than 0.5 x 10⁹/L lasting longer than 5 days
- Platelet count less than 25 x 10⁹/L
- Grade 3-4 treatment related nonhematologic toxicity except for alopecia, or reversible, transient elevations of alanine transaminase [ALT] or aspartate transaminase [AST], or nausea, and vomiting in patients who are not being given optimal antiemetic medications
- Treatment delay more than 3 weeks because of unresolved toxicity

The criteria for further dose escalation or continuation of administration of the same dose will be a function of the number of patients who have DLT as follows:

| | | |
|---|---|---|
| 0 of 3 | → | Escalate to the next dose. |
| 1 of 3 | → | Recruit 3 additional patients. |
| 1 of 6 | → | Escalate to next dose. |
| 2 of 6 | → | MTD, recommended dose (RD) is dose below MTD. |
| At least 2 of 3 or At least 3 of 6 | → | toxic: MTD is dose below the toxic dose; RD is 2 doses below. |

Duration of treatment : Each cycle is repeated every 3 weeks, unless the patient's bone marrow did not recover with a neutrophil count above 1.5 x 10⁹/L and a platelet count above 100 x 10⁹/L or nonhematologic toxicities are not resolved to grade 1. Up to 3 weeks' delay (ie, Day 42) between consecutive treatments is permitted. If the criteria allowing further treatment are not met at the end of the 21 supplemental-day period, the patient is withdrawn. Patients showing no objective disease progression (assessed every 2 cycles, for example by tumour burden, such as CAT scan or a marker measure) may continue treatment until a withdrawal criterion is met. Treatment can continue (based on a case by case decision) for as long as the investigator considers that it is in the patient's best interest in terms of disease control and general well being, unless withdrawal criteria are met.

Similar protocols may be utilised to assess the benefits of the present invention using other non-platinum based anti-cancer agents. For example, the above protocol for Gemcitabine may be adapted using the ordinary skill of a physician for use with Taxol by using an appropriate dose of Taxol (for example in the range 135-175mg/m²) and ZD0473 (for example in the range 100-150mg/m²). The above protocol may also be adapted using the ordinary skill of a physician for use with, for example, ZD0473 (1-10 mg/Kg/day, preferably 2-5 mg/Kg/day and more preferably 3-4 mg/Kg/day) and Iressa (1-20 mg/Kg/day, preferably 3-10 mg/Kg/day and more preferably 5 mg/Kg/day). Toxicity tests appropriate for the drugs being studied are used, depending on the toxicity profile expected for each drug alone.

## Claims

1. A pharmaceutical composition which comprises a sterically hindered platinum coordination compound and a non-platinum based anti-cancer agent, in association with a pharmaceutically acceptable excipient or carrier.

2. A pharmaceutical composition according to claim 1 in which the sterically hindered platinum coordination compound is (SP-4-3)-(*cis*-amminedichloro-[2-methylpyridine]platinum (II), or a pro-drug thereof.

3. A pharmaceutical composition according to claim 1 or 2 in which the non-platinum based anti-cancer agent is selected from Taxol, Gemcitabine, Navelbine, Doxil, 5-FU and Taxotere.

4. A kit comprising a sterically hindered platinum coordination compound and a non-platinum based anti-cancer agent.

5. A kit according to claim 4 in which the sterically hindered platinum coordination compound is (SP-4-3)-(*cis*-amminedichloro-[2-methylpyridine]platinum (II), or a pro-drug thereof.

6. A kit according to claim 4 or 5 in which the non-platinum based anti-cancer agent is selected from Taxol, Gemcitabine, Navelbine, Doxil, 5-FU and Taxotere.

7. A combination product comprising a sterically hindered platinum coordination compound and a non-platinum based anti-cancer agent for use as a medicament.

8. A combination product according to claim 7 in which the sterically hindered platinum coordination compound is (SP-4-3)-(*cis*-amminedichloro-[2-methylpyridine]platinum (II), or a pro-drug thereof.

9. A combination product according to claim 7 or 8 in which the non-platinum based anti-cancer agent is selected from Taxol, Gemcitabine, Navelbine, Doxil, 5-FU and Taxotere.

10. The use of a sterically hindered platinum coordination compound in combination with a non-platinum based anti-cancer agent in the manufacture of a medicament for use in the production of an anti-cancer effect in a warm-blooded animal such as a human.

11. The use according to claim 10 in which the sterically hindered platinum coordination compound is (SP-4-3)-(*cis*-amminedichloro-[2-methylpyridine]platinum (II), or a pro-drug thereof.

12. The use according to claim 10 or 11 in which the non-platinum based anti-cancer agent is selected from Taxol, Gemcitabine, Navelbine, Doxil, 5-FU and Taxotere.

13. The use according to claim 10 or 11 or 12 in which the sterically hindered platinum coordination compound and the non-platinum based anti-cancer agent are present in-vivo at the same time.

14. The use of a therapeutic combination product as defined in any of claims 7 to 9, or of a pharmaceutical composition as defined in any of claims 1 to 3 in the manufacture of a medicament for use in the production of an anti-cancer effect in a warm-blooded animal such as a human.

15. The use according to claim 14 in which the sterically hindered platinum coordination compound and the non-platinum based anti-cancer agent are present in-vivo at the same time.

16. A method for producing an anti-cancer effect in a warm-blooded animal such as a human which comprises the administration to said animal of an effective amount of a sterically hindered platinum coordination compound in combination with a non-platinum based anti-cancer agent.

17. A method for producing an anti-cancer effect in a warm-blooded animal such as a human which comprises the administration to said animal of an effective amount of a therapeutic combination product as defined in any of claims 7 to 9, or of a pharmaceutical composition as defined in any of claims 1 to 3.

18. A method according to claim 16 or 17 in which the sterically hindered platinum coordination compound and the non-platinum based anti-cancer agent are present in-vivo at the same time.
